# EUROPEAN PATENT SPECIFICATION

(11) **EP 2 877 121 B1**
(45) Date of publication and mention of the grant of the patent: **21.12.2022**
(21) Application number: 13733143.5
(22) Date of filing: 11.06.2013
(51) Int. Cl.: A61F 2/24, A61F 2/966

(54) **TRANS-AORTIC SURGICAL SYRINGE-TYPE DEVICE FOR DEPLOYMENT OF A PROSTHETIC VALVE**
CHIRURGISCHE SPRITZENARTIGE TRANSAORTENVORRICHTUNG ZUM EINSETZEN EINER KLAPPENPROTHESE
DISPOSITIF DE TYPE SERINGUE CHIRURGICALE TRANS-AORTIQUE POUR LE DÉPLOIEMENT D'UNE VALVULE PROTHÉTIQUE

(30) Priority: 25.07.2012 US 201213557505
(43) Date of publication of application: 03.06.2015
(73) Proprietor: Medtronic Vascular Galway, Galway (IE)
(72) Inventor: SHEAHAN, Edmond, Santa Rosa, California 95403 (US); ROGERS, Ronan, Santa Rosa, California 95403 (US)
(74) Representative: Zimmermann & Partner Patentanwälte mbB
(86) International application number: PCT/US2013/045229
(87) International publication number: WO 2014/018173

(56) References cited:
- WO-A1-01/34061
- WO-A1-96/37167
- WO-A2-03/003944
- WO-A2-2011/008812
- US-A1- 2003 060 777
- US-A1- 2010 174 363
- US-A1- 2011 224 678

## Description

### BACKGROUND

### Field

The present disclosure relates to systems for delivering a prosthesis to a desired location in the body of a patient and discloses methods for delivering and implanting a prosthesis. More particularly, the present disclosure relates to prosthetic valve introducer systems for deploying a heart valve prosthesis within a body lumen and to methods of delivering such a prosthesis to a desired location in the body,

### Background

Currently, replacement of a deficient cardiac valve is often performed by placing the patient under extracorporeal circulation, temporarily stopping the heart, opening the thorax (e.g., by a sternotomy), surgically opening the heart, excising the deficient valve, and then implanting a prosthetic valve in its place. This procedure generally requires prolonged patient hospitalization, as well as extensive and often painful recovery.

Recently, minimally invasive approaches have been developed to facilitate catheter-based implantation of valve prostheses in the beating heart, intending to obviate the need for the classic sternotomy and cardiopulmonary bypass. For example, U.S. Patent No. 8,016,877 to Seguin et al*.* illustrates a technique and a device for replacing a deficient heart valve by percutaneous route. An expandable prosthetic valve can be compressed about a catheter, inserted inside a body lumen, such as the femoral artery, and delivered to a desired location in the heart. Additionally, U.S. Patent No. 7,914,569 to Nguyen et al. discloses advancing a catheter containing a prosthesis in a retrograde manner through the femoral artery and into the descending aorta, over the aortic arch, through the ascending aorta and inside the defective aortic valve. This procedure can be assisted by fluoroscopic guidance. Once the position of the catheter containing the prosthesis is confirmed, a sheath containing the prosthesis can be moved proximally, allowing the valve prosthesis to self-expand.

However, in certain instances it may still be necessary to use the classic sternotomy technique. It would be desirable to avoid cardiopulmonary bypass during this procedure while still obtaining accurate positioning of the prosthetic valve,

Other techniques for delivering prosthetic heart valves via a catheter include transapical and trans-aortic approaches for aortic valve replacement, typically involving the use of an introducer port, i.e., a large-bore overtube, of a trocar. A crimped, framed valve prosthesis reversibly coupled to a delivery catheter can be transcatheterally advanced toward the native valve, where it can either be deployed using a balloon catheter, or, alternatively, using a self-expandable system.

With regard to the structure of the heart valve prosthesis itself, U.S. Patent No. 7,914,569 to Nguyen et al. describes an example prosthesis for percutaneous transluminal delivery, The heart valve prosthesis can have a self-expanding multi-level frame that supports a valve body with a skirt and plurality of leaflets. The frame can be contracted during percutaneous transluminal delivery and expanded to an hourglass shape upon deployment within the native heart valve.

### BRIEF SUMMARY

The present disclosure describes valve introducer systems for implanting a heart valve prosthesis through a trans-aortic pathway. By directly accessing the aorta, it is possible to accurately implant a prosthetic valve without the need for cardiopulmonary bypass, although the disclosed valve introducer systems can also be used with cardiopulmonary bypass. Furthermore, the valve introducer systems disclosed herein allow for one-handed deployment of the prosthetic valve, providing a quick, single-shot and accurate deployment.

The valve introducer can include a delivery shaft with a distal tip and a tubular member to house a prosthetic valve, and a deployment element to push the prosthetic valve out of the distal tip of the delivery shaft. The valve introducer includes a handle with a deployment element, a deployment element housing and at least one finger gripping element connected to the deployment element housing.

In order to deliver the valve prosthesis, the delivery shaft can be inserted into a body lumen and advanced to a desired deployment location. The prosthetic valve can be deployed by pushing the deployment element in a distal direction, thereby pushing the prosthetic valve out the distal tip of the delivery shaft.

### BRIEF DESCRIPTION OF THE DRAWINGS

The accompanying figures, which are incorporated herein, form part of the specification and illustrate valve introducer systems and methods of delivering a prosthesis to a desired location in a body of a patient. Together with the description, the figures further serve to explain the principles of and allow for the making and using of the valve introducer systems and methods described herein. These figures are intended to be illustrative, not limiting. Although the disclosure is generally described in the context of these embodiments, it should be understood that it is not intended to limit the scope of the disclosure to these particular embodiments. In the drawings, like reference numbers indicate identical or functionally similar elements.
FIG. 1 illustrates a valve introducer system including a deployment element housing and a capsule to house a prosthetic valve, according to an embodiment.
FIG. 2 illustrates a close-up view of a handle assembly, according to an embodiment.
FIG. 3 illustrates a valve introducer system, that is not claimed.
FIG. 4 illustrates a valve introducer system inserted into an aorta prior to deployment of a heart valve prosthesis, that is not claimed.
FIG. 5 illustrates a valve introducer system, including a hemostatic valve, inserted into an aorta after deployment of a heart valve prosthesis, that is not claimed

### DETAILED DESCRIPTION

While the disclosure refers to illustrative embodiments for particular applications, it should be understood that the disclosure is not limited thereto. Modifications can be made to the embodiments described herein without departing from the scope of the present disclosure. Those skilled in the art with access to this disclosure will recognize additional modifications, applications, and embodiments within the scope of this disclosure and additional fields in which the disclosed examples could be applied. Therefore, the following detailed description is not meant to be limiting. Further, it is understood that the systems and methods described below can be implemented in many different embodiments of hardware. Any actual hardware described is not meant to be limiting. The operation and behavior of the systems and methods presented are described with the understanding that modifications and variations of the embodiments are possible given the level of detail presented.
The invention is defined in claim 1. Preferred embodiments are defined in the dependent claims.

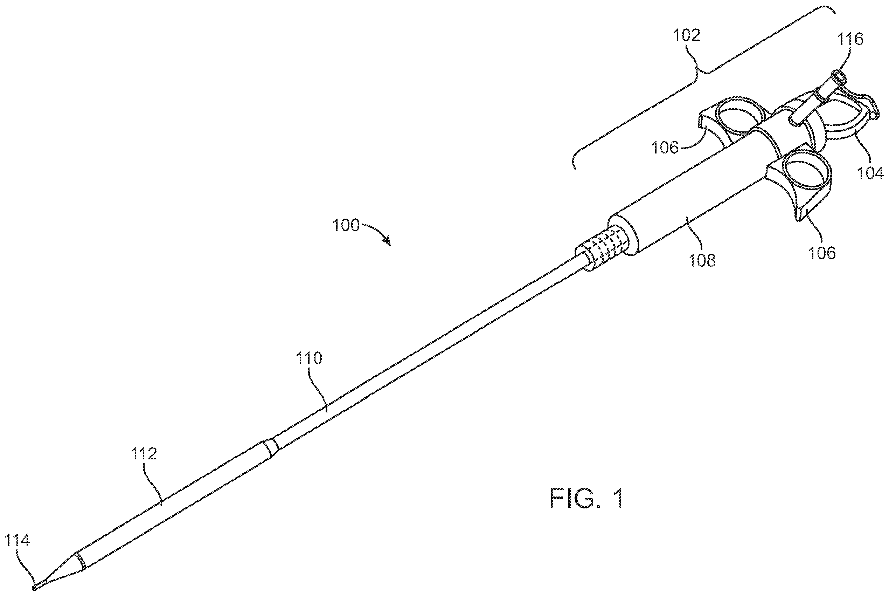

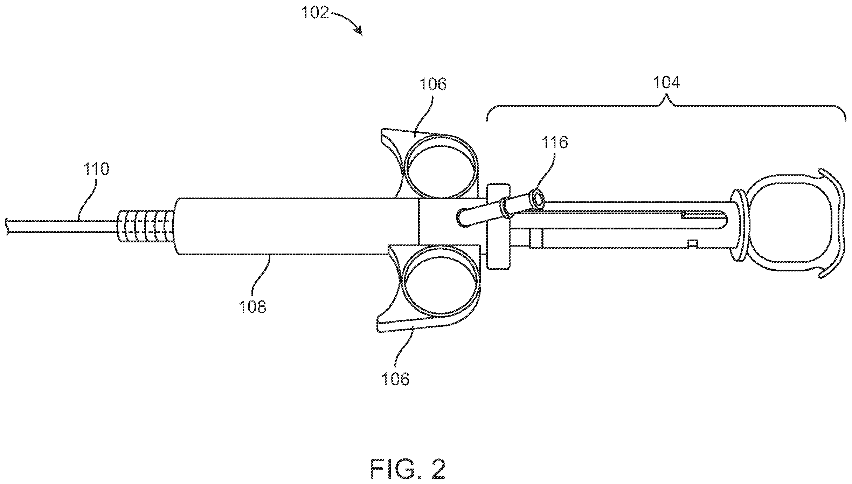

FIG. 1 illustrates an embodiment of delivery device 100, which generally includes handle 102, delivery shaft 110, capsule 112, and distal tip 114. FIG. 2 shows a close up view of handle 102, which includes deployment element 104, deployment element housing 108, and finger grips 106. Handle 102 can also include access port 116. In certain embodiments, parts of delivery device 100 can be made from biocompatible materials, such as certain biocompatible polymers and metals known in the art.

Deployment element 104 is used to deploy a prosthetic valve loaded into delivery device 100. Generally, deployment element 104 can be designed to deploy the prosthetic valve in a single shot, that is, by deploying the prosthetic valve from delivery device 100 in one smooth motion. The user can control the speed of deployment by the rate at which the user activates deployment element 104. In certain instances, the user may wish to reposition the prosthetic valve after beginning to deploy the prosthetic valve. The user can accomplish this by moving the entire delivery device 100 in a distal or proximal direction.

In certain embodiments, deployment element 104 and deployment element housing 108 can be generally cylindrical in shape. Deployment element 104 is configured to slidably engage with deployment element housing 108, such as by a channel or groove, as illustrated in FIG. 2. The circumference of deployment element 104 can also be such that it frictionally engages deployment element housing 108. This can provide controlled movement of deployment element 104 during deployment of the prosthetic valve. Deployment element 104 can be made of materials such as plastic or rubber to impart enough force to push the prosthetic valve out of distal tip 114, but without damaging the structural integrity of the prosthetic valve. In embodiments where the distal end of deployment element 104 contacts the prosthetic valve, the distal end of deployment element 104 can be made of a softer material to prevent damaging the prosthetic valve during deployment.

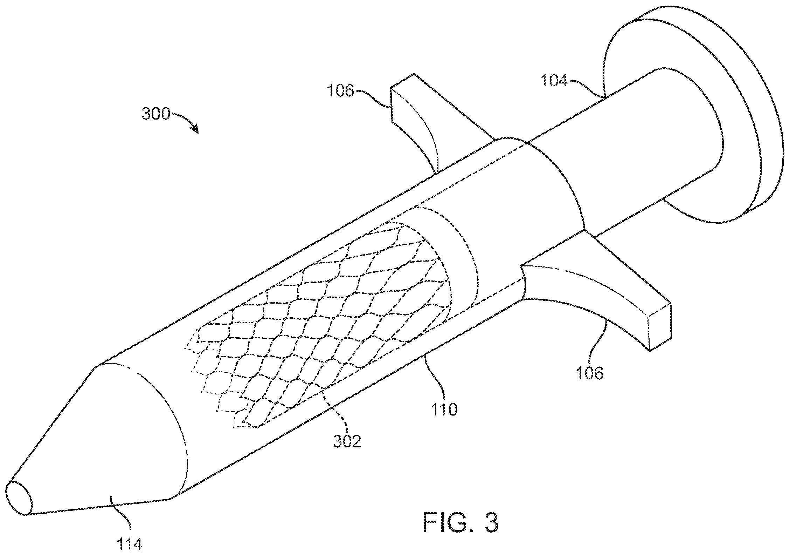

In certain embodiments, deployment element 104 can include a feature to assist in pushing or pulling deployment element 104. For example, FIG. 2 illustrates a hole or ring-shaped feature located at the proximal end of deployment element 104 to allow for insertion of a finger. This feature can also be saddle-shaped, or simply a flat surface, as shown in FIG. 3. This feature can aid in deploying the prosthetic valve by providing tactile feel and preventing the user's finger from slipping from deployment element 104. Generally, the thumb can be used to push or pull deployment element 104 to deploy the prosthetic valve.

One or more finger grips 106 are connected to, deployment element housing 108. Finger grips 106 include an interior surface formed by a hole or ring element to allow insertion of a finger, and an exterior surface for gripping. The gripping surfaces are curved to provide comfort and tactile feel. Generally, the index finger and middle finger can utilize finger grips 106 to steady delivery device 100 while the thumb can be used to push or pull deployment element 104 during deployment of the prosthetic valve. By grasping finger grips 106 and pushing or pulling deployment element 104 with the thumb, the user can deploy the prosthetic valve.

Handle 102 can also include access port 116. Access port 116 can be used, for example, to extract air from the system, introduce fluid into the system, or insert additional surgical tools through the system and into the body lumen.

Delivery shaft 110 can be inserted into a body lumen to deliver a prosthetic valve. In certain embodiments, delivery shaft 110 can be generally cylindrical in shape, with a smooth exterior surface. This can help prevent damage to the interior of the body lumen. Capsule 112 and distal tip 114 can be located at the distal end of delivery shaft 110. Capsule 112 can house a prosthetic valve. Generally, a collapsible and self-expanding prosthetic valve, such as the one in U.S. Patent No. 7,914,569 to Nguyen et al.*,* can be loaded into capsule 112 prior to the surgical procedure.

The interior of delivery shaft 110, capsule 112 and distal tip 114 can all be coated with a biocompatible lubricant, which can decrease friction with the prosthetic valve and allow a smooth exit during deployment. Distal tip 114, is an atraumatic tip. It is flexible to prevent damage to the interior of the body lumen, and expandable to allow it to expand from its generally tapered configuration according to the invention where the prosthetic valve is pushed out of delivery shaft 110 during deployment. Distal tip 114 is biased to a tapered configuration so that it returns to the tapered configuration after the prosthetic valve is deployed out of distal tip 114. In certain embodiments, distal tip 114 can be radiopaque to assist in locating distal tip 114 with medical imaging during the implantation procedure. In certain embodiments, distal tip 114 can be used to puncture the exterior surface of a body lumen to provide access to the interior of the body lumen, similar to a syringe.

Deployment element 104 has a portion that extends through the interior of delivery shaft 110 that is capable of pushing the prosthetic valve out of capsule 112 and through distal tip 114 in order to deploy the prosthetic valve, In certain embodiments, deployment element 104 can also be in contact with a pushing element that extends from deployment element housing 108 through the interior of delivery shaft 110 to capsule 112 in order to push the prosthetic valve out of capsule 112 and through distal tip 114.

FIG. 3 illustrates a prosthetic valve delivery system that is not claimed. Prosthetic valve 302 can be housed directly within delivery shaft 110 of delivery device 300. By pushing deployment element 104 in a distal direction, prosthetic valve 302 can be forced out of distal tip 114. The interior of delivery shaft 110 and distal tip 114 can be coated with a biocompatible lubricant to decrease friction with the prosthetic valve and allow a smooth exit during deployment. Finger grips 106 can also be provided to assist in gripping delivery device 300. In FIG. 3, deployment element 104 has a flat surface at its proximal end. As discussed above, this feature can take other forms, such as a saddle-shaped or ring-shaped feature. This can provide a stable surface to push when deploying prosthetic valve 302. The distal end of deployment element 104 can be in contact with the proximal end of prosthetic valve 302, such that by pushing deployment element 104 in a distal direction, prosthetic valve 302 also moves in the distal direction. By depressing deployment element 104, prosthetic valve 302 can be pushed out of delivery device 300 through distal tip 114.

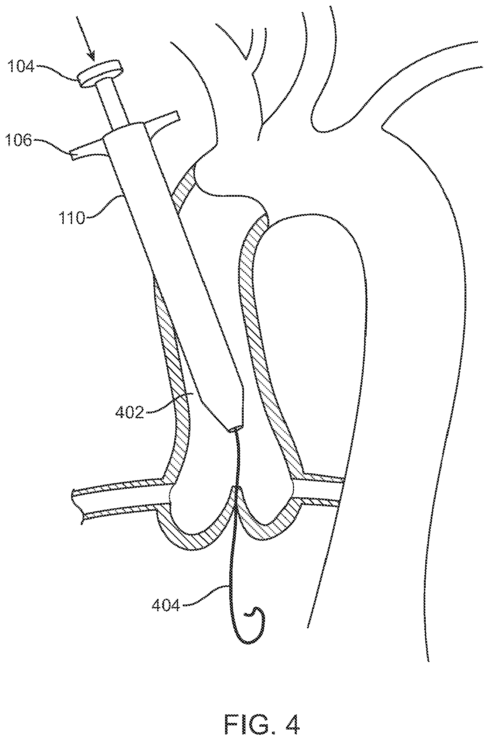

FIG. 4 illustrates delivery device 300 that is not claimed inserted into aorta 402. For trans-aortic delivery of a prosthetic aortic valve, delivery device 300 can generally be inserted into the ascending aorta. Delivery device 300 can then be advanced toward the aortic sinus, which is generally the deployment location for a prosthetic aortic valve. Guide wire 404 can be included as part of the delivery system to help direct delivery shaft 110 of delivery device 300 to the desired deployment location. Once in the desired location, deployment element 104 can be pushed in the distal direction, as indicated by the arrow

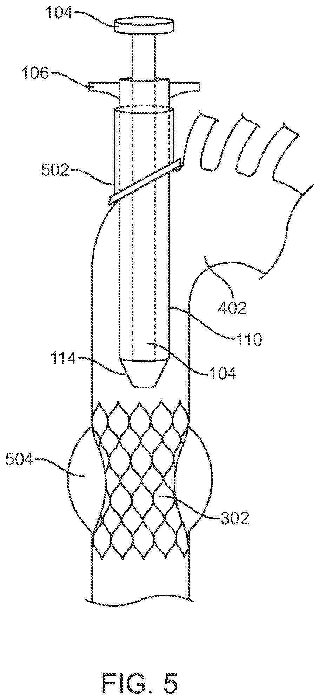

FIG. 5 illustrates delivery device 300 that is not claimed after deploying prosthetic valve 302. Generally, prosthetic valve 302 is deployed within aortic sinus 504. As shown in FIG. 5, delivery device 300 can be coupled with hemostatic valve 502. Delivery device 300 can be introduced into aorta 402 by inserting delivery shaft 110 through hemostatic valve 502. Hemostatic valve 502 can be sutured in place to aorta 402 to provide a stable access point for delivery device 300. Hemostatic valve 502 can also contain any bleeding that may occur during the valve implantation procedure.

Methods of delivering a prosthetic valve are also disclosed but not claimed. Reference to the Figures may be used by way of example. Prior to surgery, the desired valve implantation location should be determined. This can be done with the assistance of medical imaging, such as a CT scan. For prosthetic aortic valve implantation via a trans-aortic route, the implantation location is generally located within aortic sinus 504 such that the distal part of prosthetic valve 302 engages the leaflets of the natural aortic valve, and the proximal part of prosthetic valve 302 engages the inner wall of the ascending aorta. Alternative implantation sites can be used, and the optimal implantation site can be determined by a physician for each individual patient.

Generally, a patient's chest can be opened such that aorta 402 is exposed. This can be accomplished, for example, by a mini-sternotomy or a thoracotomy. Delivery shaft 110 can be inserted into aorta 402 at an insertion point. The aorta, as defined herein, can include the exterior surface and lumen of the descending aorta, aortic arch, ascending aorta and aortic sinus. In certain embodiments, distal tip 114 of delivery shaft 110 can be used to puncture the surface of aorta 402 at the insertion point. In certain embodiments, an incision can be made and hemostatic valve 502 can be secured at the insertion point to provide an access point to aorta 402.

A radially collapsed prosthetic valve 302 can be loaded into delivery shaft 110 or capsule 112. In certain methods, prosthetic valve 302 can be pre-loaded in delivery device 100 or 300. Delivery shaft 110 can then be inserted directly into aorta 402, or through hemostatic valve 502 into aorta 402.

After delivery shaft 110 is inserted into aorta 402, delivery shaft 110 can be advanced distally until distal tip 114 reaches the desired deployment location. In certain methods medical imaging can be used to locate distal tip 114 of delivery shaft 110 within aorta 402 prior to deployment of prosthetic valve 302. Delivery shaft 110 can be moved in a proximal or distal direction to adjust the deployment location of prosthetic valve 302 so that prosthetic valve 302 can be properly implanted within aortic sinus 504.

Once in the proper location, in certain embodiments, prosthetic valve 302 can be deployed by pushing deployment element 104 in a distal direction, thereby pushing

Generally, prosthetic valve 302 can be a self-expanding prosthetic valve, such that it will expand to a pre-fabricated size and shape within aortic sinus 504 after being deployed. In certain methods, a balloon can be included under the prosthetic valve, and inflated to expand and deploy the prosthetic valve.

After deployment of prosthetic valve 302, delivery device 300 can be removed from aorta 402. If hemostatic valve 502 is used, it can be detached from aorta 402, and sutures can be used to close the insertion point.

## Claims

1. A prosthetic valve introducer system, the valve introducer comprising:
a handle (102) comprising:
a deployment element housing (108),
a deployment element (104), wherein the deployment element comprises a shaft with a proximal end, wherein the shaft is slidably engaged with an interior surface of the deployment element housing, and
at least one finger gripping element (106) connected to the deployment element housing (104) and comprising an interior gripping surface and an exterior gripping surface, the interior gripping surface configured to allow insertion of a finger, the gripping surfaces being curved to provide comfort and tactile feel; and
a delivery shaft (110) comprising a tubular member,
a prosthetic valve (302), a housing capsule (112) for the prosthetic valve (302), and an atraumatic distal tip (114), biased to a tapered configuration, the distal tip (114) being flexible to prevent damage to the interior of a body lumen, and expandable, from the tapered configuration, to allow for pushing the prosthetic valve (302) out of the delivery shaft during deployment; and
wherein the deployment element (104) has a portion that extends through the interior of the delivery shaft (110) that is capable of pushing the prosthetic valve (302) out of capsule (112) and through the distal tip (114) in order to deploy the prosthetic valve (302) from the delivery shaft (110),
wherein the delivery shaft (110) is configured such that the delivery shaft can be advanced until distal tip (114) reaches the desired deployment location,
and the system is configured such that the prosthetic valve is deployed by pushing the deployment element in a distal direction, thereby pushing the prosthetic valve out of the distal tip of the delivery shaft.

2. The prosthetic valve introducer system of claim 1, wherein the proximal end of the deployment element comprises a flat surface.

3. The prosthetic valve introducer system of claim 1, further comprising a hemostatic valve (502) configured to provide access to a body lumen.

4. The prosthetic valve introducer system of claim 1, wherein the handle further comprises an access port.

5. The prosthetic valve introducer system of claim 1, wherein the prosthetic valve is a self-expanding prosthetic heart valve.

## Patentansprüche

1. Einführungssystem für eine Klappenprothese, das Klappeneinführungssystem umfassend:
einen Griff (102), umfassend:
ein Einsetzelementgehäuse (108),
ein Einsetzelement (104), wobei das Einsetzelement ein Welle mit einem proximalen Ende umfasst, wobei die Welle mit einer Innenoberfläche des Einsetzelements verschiebbar in Eingriff steht, und
mindestens ein Fingergreifelement (106), das mit dem Einsetzelementgehäuse (104) verbunden ist und umfassend eine Innengreifoberfläche und eine Außengreifoberfläche, wobei die Innengreifoberfläche konfiguriert ist, um ein Einschieben eines Fingers zu ermöglichen, wobei die Greifoberflächen gekrümmt sind, um Komfort und taktiles Gefühl bereitzustellen; und
eine Abgabewelle (110), umfassend ein rohrförmiges Element,
eine Klappenprothese (302), eine Gehäusekapsel (112) für die Klappenprothese (302) und eine atraumatische distale Spitze (114), die auf eine verjüngte Konfiguration vorgespannt ist, wobei die distale Spitze (114) flexibel ist, um Schäden an dem Inneren eines Körperlumens zu verhindern und aus der verjüngten Konfiguration expandierbar zu sein, um ein Drücken der Klappenprothese (302) aus der Abgabewelle während eines Einsetzens zu ermöglichen; und
wobei das Einsetzelement (104) einen Abschnitt aufweist, der sich durch das Innere der Abgabewelle (110) erstreckt, die in der Lage ist, die Klappenprothese (302) aus der Kapsel (112) und durch die distale Spitze (114) zu drücken, um die Klappenprothese (302) aus der Abgabewelle (110) einzusetzen,
wobei die Abgabewelle (110) derart konfiguriert ist, dass die Abgabewelle vorgeschoben werden kann, bis die distale Spitze (114) die gewünschte Einsetzstelle erreicht,
und das System derart konfiguriert ist, dass die Klappenprothese mittels Drückens des Einsetzelements in einer distalen Richtung, eingesetzt wird, wobei dadurch die Klappenprothese aus der distalen Spitze der Abgabewelle gedrückt wird.

2. Einführungssystem für eine Klappenprothese nach Anspruch 1, wobei das proximale Ende des Einsetzelements eine flache Oberfläche umfasst.

3. Einführungssystem für eine Klappenprothese nach Anspruch 1, ferner umfassend eine hämostatisches Klappe (502), die konfiguriert ist, um Zugang zu einem Körperlumen bereitzustellen.

4. Einführungssystem für eine Klappenprothese nach Anspruch 1, wobei der Griff ferner einen Zugangsanschluss umfasst.

5. Einführungssystem für eine Klappenprothese nach Anspruch 1, wobei die Klappenprothese eine selbstexpandierende Herzklappenprothese ist.

## Revendications

1. Système d'introduction de valve prothétique, le dispositif d'introduction de valve comprenant :
une poignée (102) comprenant :
un logement d'élément de déploiement (108),
un élément de déploiement (104), l'élément de déploiement comprenant un arbre avec une extrémité proximale, l'arbre étant mis en prise de manière coulissante avec une surface intérieure du logement de l'élément de déploiement, et
au moins un élément de préhension de doigt (106) relié au logement d'élément de déploiement (104) et comprenant une surface de préhension intérieure et une surface de préhension extérieure, la surface de préhension intérieure étant conçue pour permettre l'insertion d'un doigt, les surfaces de préhension étant incurvées pour fournir un confort et un mouvement tactile ; et
un arbre d'administration (110) comprenant un élément tubulaire,
une valve prothétique (302), une capsule de logement (112) pour la valve prothétique (302), et une pointe distale atraumatique (114), sollicitée à une configuration effilée, la pointe distale (114) étant flexible pour empêcher un dommage à l'intérieur d'une lumière corporelle, et expansible, à partir de la configuration effilée,pour permettre de pousser la valve prothétique (302) hors de l'arbre d'administration pendant le déploiement ; et
l'élément de déploiement (104) ayant une partie qui s'étend à travers l'intérieur de l'arbre d'administration (110) qui est capable de pousser la valve prothétique (302) hors de la capsule (112) et à travers la pointe distale (114) afin de déployer la valve prothétique (302) à partir de l'arbre d'administration (110),
l'arbre d'administration (110) étant conçu de telle sorte que l'arbre d'administration peut êtreavancé jusqu'à ce que la pointe distale (114) atteint l'emplacement de déploiement souhaité,
et le système étant conçu de telle sorte que la valve prothétique est déployée en poussant l'élément de déploiement dans une direction distale, en poussant ainsi la valve prothétique hors de la pointe distale de l'arbre d'administration.

2. Système d'introduction de valve prothétique selon la revendication 1, dans lequel l'extrémité proximale de l'élément de déploiement comprend une surface plate.

3. Système d'introduction de valve prothétique selon la revendication 1, comprenant en outre une valve hémostatique (502) conçue pour fournir un accès à une lumière corporelle.

4. Système d'introduction de valve prothétique selon la revendication 1, dans lequel la poignée comprend en outre un orifice d'accès.

5. Système d'introduction de valve prothétique selon la revendication 1, dans lequel la valve prothétique est une valve cardiaque prothétique auto-expansible.
